# FASCICULE DE BREVET EUROPEEN

(11) **EP 3 089 981 B1**
(45) Date de publication et mention de la délivrance du brevet: **19.09.2018**
(21) Numéro de dépôt: 14833151.5
(22) Date de dépôt: 29.12.2014
(51) Int. Cl.: C07D 491/056

(54) **CHLORHYDRATE DE TRITOQUALINE SOUS FORME CRISTALLISEE ET SON PROCEDE D'OBTENTION**
TRITOQUALINHYDROCHLORID IN KRISTALLISIERTER FORM UND SEIN HERSTELLUNGSVERFAHREN
TRITOQUALINE HYDROCHLORIDE IN CRYSTALLIZED FORM AND ITS PREPARATION PROCESS

(30) Priorité: 30.12.2013 FR 1303107
(43) Date de publication de la demande: 09.11.2016
(73) Titulaire: Binay, Patrice, 21160 Couchey (FR)
(72) Inventeur: Binay, Patrice, 21160 Couchey (FR)
(74) Mandataire: Gallochat, Alain
(86) Numéro de dépôt international: PCT/FR2014/000306
(87) Numéro de publication internationale: WO 2015/101727

(56) Documents cités:
- WO-A1-2013/164204
- GB-A- 873 935
- C. MASLINSKI ET AL: "Tritoqualine and some aspects of histamine metabolism", ARZNEIMITTEL-FORSCHUNG/DRUG RESEARCH, vol. 36, no. 5, 1986, pages 822-825, XP001526273,

## Description

La présente invention concerne une forme cristallisée de chlorhydrate de tritoqualine ainsi qu'un procédé d'obtention.

La tritoqualine, dont le nom chimique est la 7-amino-4,5,6-triéthoxy-3-(4-méthoxy-6-méthyl-7,8-dihydro-5H-[1,3]dioxolo[4,5-g]isoquinolin-5-yl)-3H-isobenzofuran-1-one est une molécule connue pour son action antihistaminique supposée ; c'est en particulier un inhibiteur enzymatique de la L-histidine décarboxylase. Il a été démontré depuis peu que cette activité était très faible ; en revanche son activité sur le récepteur H4 suscite un vif intérêt depuis quelques années. En particulier cette molécule a été largement étudiée dans le domaine des maladies inflammatoires digestives et plus particulièrement gastriques et oesophagiennes.

Il a également été trouvé que la tritoqualine avait un intérêt dans le traitement des leucémies aigües et dans celui de la mucoviscidose, la broncho-pneumopathie chronique obstructive (BPCO) et de l'exacerbation de l'asthme.

Ces diverses applications de la tritoqualine ont fait l'objet de nombreux brevets.

Toutefois, l'utilisation optimale de la tritoqualine s'est heurtée à différents problèmes liés aux propriétés physico chimiques intrinsèques de la molécule, et en particulier à sa très faible solubilité (100 ppm dans l'eau à température ambiante).

Différentes méthodes ont été utilisées pour accroître la solubilité et par conséquent la biodisponibilité de la tritoqualine, par exemple en passant par le stade du chlorhydrate de tritoqualine formé in situ par adjonction d'acide chlorhydrique à de la tritoqualine en solution. Celui-ci a une durée de demi-vie de quelques heures en solution aqueuse. La molécule se coupe au niveau des 2 carbones asymétriques entre le cycle isoquinoléine (cotamine) et le cycle phtalique.

Malgré de nombreux essais, il n'a jamais été possible d'isoler le chlorhydrate de tritoqualine sous forme cristallisée, ce qui aurait permis une plus grande reproductibilité des études cliniques menées avec cette molécule et une meilleure maîtrise de l'action de la tritoqualine lors de son administration aux patients.

La présente invention concerne précisément le chlorhydrate de tritoqualine sous forme cristallisée ainsi qu'un procédé d'obtention de cette forme cristallisée.

Un procédé préférentiel de synthèse du chlorhydrate cristallisé de tritoqualine va maintenant être décrit, la réaction étant globalement la suivante :

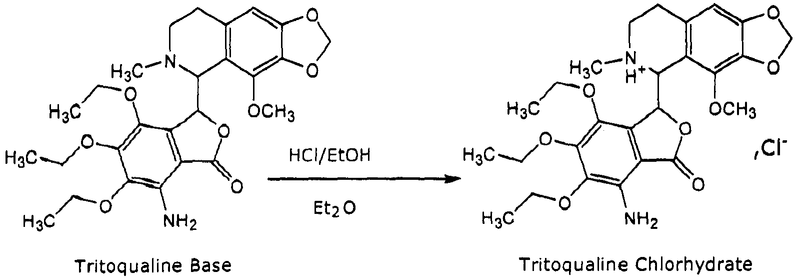

Un mode opératoire va maintenant être décrit plus en détails.

Dans un erlenmeyer de 500 ml muni d'un barreau aimanté et d'une agitation magnétique, introduire à l'aide d'une spatule 30 g de tritoqualine base dans 45 ml d'éthanol absolu (1,5 volume) à température ambiante, puis ajouter rapidement et sous agitation rapide 6 ml d'acide chlorhydrique à 37 % (soit 1 équivalent molaire).

Dès la fin de l'ajout de l'acide chlorhydrique, la tritoqualine base encore en suspension, se dissout progressivement dans l'éthanol en quelques minutes. Bien que cette solution soit très concentrée (solution sursaturée), le chlorhydrate de tritoqualine reste en solution en raison d'un phénomène de surfusion. Ajouter progressivement de l'ordre de 120 ml d'éther éthylique (4 volumes) par fraction successive de 15 à 20 ml. Chaque ajout provoque un trouble de la solution par apparition d'une seconde phase. Cette opalescence disparaît au bout de quelques dizaines de secondes d'agitation à température ambiante et la solution redevient claire et translucide. Poursuivre l'ajout de petites fractions d'éther éthylique. Lorsque les ajouts cumulés d'éther sont de l'ordre de 3 volumes, l'opalescence devient persistante, stopper l'ajout d'éther à ce moment précis et maintenir l'agitation pendant 15 mn à température ambiante ; le chlorhydrate de tritoqualine précipite progressivement durant cette phase. Ajouter le reliquat d'éther (à concurrence de 4 volumes) afin de parfaire le rendement de cristallisation. Maintenir l'agitation pendant une demi-heure supplémentaire. L'agitation doit être suffisante pour permettre d'obtenir une suspension d'apparence homogène.

Si l'agitation est trop faible, le chlorhydrate de tritoqualine décante dans le fond de l'erlenmeyer ; a contrario, si l'ajout de l'éther est trop rapide (c'est-à-dire sans attendre que l'opalescence ne disparaisse entre deux ajouts d'éther, alors le chlorhydrate de tritoqualine est de très mauvaise qualité : il ne précipite pas convenablement et conduit à un produit plus ou moins amorphe et collant (une sorte de gel incolore et visqueux gorgé de solvant) ou un mélange de cristaux et de gel amorphe très collant.

Lorsque l'opération de cristallisation est mené convenablement selon le procédé ci-dessus, la suspension obtenue est filtrée sous pression réduite sur verre fritté n°3 ou 4 ; la filtration est rapide et le gâteau ne colmate pas le filtre.

Le gâteau est d'aspect très blanc et les eaux mères sont incolores à légèrement jaunes. Il est néanmoins préférable de procéder à un lavage piston du gâteau avec 20 ml (2 volumes) d'éther éthylique supplémentaires.

Les jus de filtration et les jus de lavage sont réunis et remis sous agitation pendant 1 heure supplémentaire en refroidissant progressivement l'ensemble à 5 °C. Un second jet précipite à nouveau. Celui-ci est filtré puis est traité de la même façon que le premier jet de cristallisation.
Masse du 1^{er} jet après séchage : 28,0 g soit un rendement de 87 %
Masse du 2^{eme} jet après séchage : 3.6 g soit au total 31.6 g de chlorhydrate cristallisé de tritoqualine.
Masse théorique attendue : 32,3 g / Rendement global = 98 %

Les propriétés physico chimiques comparatives de la tritoqualine base et du chlorhydrate cristallisé de tritoqualine sont réunies dans le tableau ci-dessous.

| Tritoqualine | PF °C | Solubilité | | Stabilité à la lumière |
|---|---|---|---|---|
| | | dans l'eau | dans l'éthanol | |
| Base C₂₆H₃₂N₂O₈ Masse molaire : 500.5 | 184°C | 150 ppm (pratiquement insoluble et hydrophobe) | 2.5 g/l à 20°C, | Jaunit progressivement après quelques jours d'exposition à la lumière naturelle |
| | | | 50 g/l au reflux de l'éthanol | |
| Chlorhydrate C₂₆H₃₃Cl N₂O₈ Masse molaire : 537 | Décomposition à T > 170°C | hydrophile et soluble à pH <3. se décompose progressivement en solution aqueuse lorsque la température augmente | > 300 g/l à 20°C, se décompose au reflux en cotarnine | Stable à la lumière naturelle pendant plusieurs semaines. |

Par ailleurs, le chlorhydrate de tritoqualine est très peu soluble voire insoluble dans les éthers ; il est en revanche soluble dans les alcools primaires, l'acétone et la méthyl éthyl cétone.

Différentes analyses ont été menées afin d'établir que le produit formé est bien le chlorhydrate de tritoqualine.

Ainsi, le spectre IR du chlorhydrate de tritoqualine illustré dans le graphe ci-dessous fait bien apparaître une bande large à 2400 cm⁻¹ caractéristique du groupe ammonium.

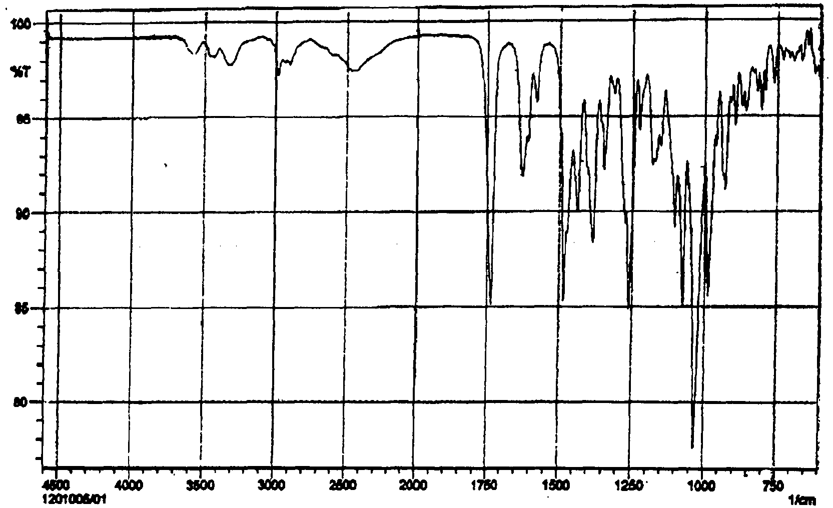

En revanche cette bande n'apparaît pas sur le spectre IR de la tritoqualine base illustré dans le graphe ci-dessous.

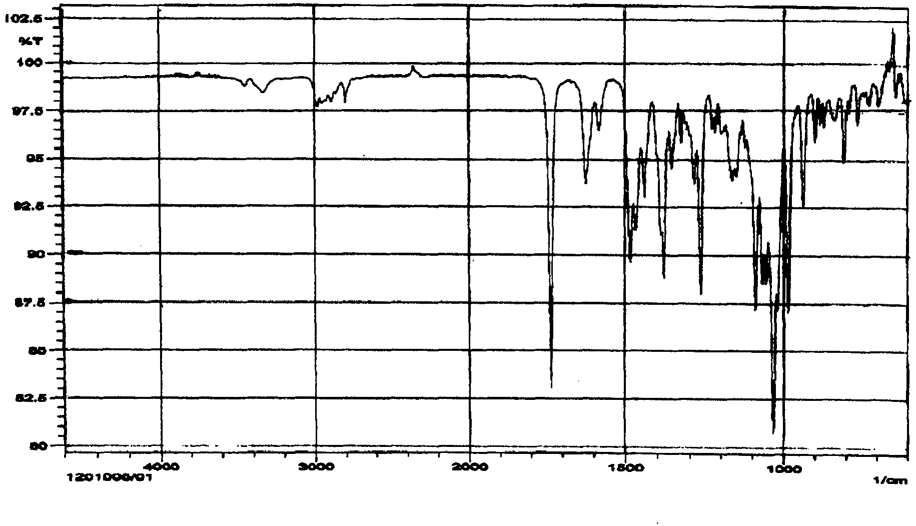

Le spectre RMN ¹H et ¹³C. non représenté ici, confirme bien qu'il s'agit du chlorhydrate de tritoqualine.

Par ailleurs, le dosage des ions chlorures est de 6,7 % et correspond à la formation d'un monochlorhydrate. La synthèse d'un dichlorhydrate (un sur l'azote de la quinoléine et un second sur l'amine primaire) a été tentée en ajoutant deux équivalents d'acide chlorhydrique. On obtient toujours le monochlorhydrate identique à celui précédemment décrit.

Enfin, il est intéressant de noter que le chlorhydrate de tritoqualine présente le même profil d'impuretés que celui de la tritoqualine base utilisée pour sa synthèse.

Dans l'exemple préférentiel de synthèse précédemment décrit, l'éthanol est utilisé comme solvant, mais d'autres alcools pourraient être utilisés, en particulier les alcools aliphatiques inférieurs, par exemple le méthanol ou l'isopropanol. Bien que donnant des rendements moins élevés et une cristallisation plus aléatoire, il a également été possible de dissoudre la tritoqualine base en présence d'acide chlorhydrique dans l'acétone ou la méthyl éthyl cétone, ou bien encore dans des dérivés glycolés tel que le glyme, également dénommé diméthyl glycol, le diglyme, également dénommé di(2-méthoxyéthyl)éther, ou le diéthylène glycol monométhyl éther.

De même l'éther éthylique utilisé lors de la cristallisation du chlorhydrate de tritoqualine pourrait être remplacé par un autre éther, comme par exemple le ter-butyl méthyl éther ou le tétrahydrofurane.

Le chlorhydrate de tritoqualine ainsi obtenu est hydrophile et soluble en solution aqueuse à pH < 3,0 comme cela apparaît dans le tableau ci-dessus ; cette solubilité renforce l'action de la tritoqualine dans l'estomac - dont le contenu a un pH de l'ordre de 2 à 3,5 - dans le traitement des maladies inflammatoires digestives. Lorsque la solution aqueuse atteint un pH >3,0 le chlorhydrate de tritoqualine précipite, mais, de façon tout à fait intéressante, cette précipitation se fait sous forme de très fines particules, donnant alors à la solution l'aspect d'un lait et augmentant ainsi de façon substantielle la biodisponibilité de la tritoqualine qui voit sa surface de contact considérablement augmentée.

On comprendra mieux ainsi l'importance d'une forme cristallisée de chlorhydrate de tritoqualine qui, contrairement à la tritoqualine base qui est hydrophobe et pratiquement insoluble, est à la fois hydrophile et soluble.

Il est apparu en outre, de façon tout à fait inattendue, qu'il existe une différence très importante de solubilité dans les alcools et les glycols entre la tritoqualine base et le chlorhydrate de tritoqualine. ce dernier étant beaucoup plus soluble que la tritoqualine base.

Cette solubilité accrue du chlorhydrate de tritoqualine dans les alcools et les glycols permet ainsi d'accéder à des formulations inaccessibles par un autre moyen.

Par ailleurs, le chlorhydrate de tritoqualine est très stable comme cela ressort du tableau ci-dessus. Cette stabilité tout à fait inattendue par rapport à la faible stabilité à la lumière de la tritoqualine base permet de préparer des compositions pharmaceutiques contenant la tritoqualine sous forme de son chlorhydrate pour les usages thérapeutiques précédemment cités.

Dans le mode opératoire précédemment décrit, on utilise l'acide chlorhydrique concentré ; le chlorhydrate de tritoqualine peut également être obtenu dans des conditions satisfaisantes en utilisant l'acide chlorhydrique sous forme gazeuse injectée dans la solution.

## Revendications

1. Chlorhydrate de tritoqualine **caractérisé en ce qu'**il se présente sous forme cristallisée.

2. Chlorhydrate selon la revendication 1 **caractérisé en ce qu'**il est soluble en solution aqueuse et hydrophile à pH < 3,0 et qu'il précipite sous forme de très fines particules à pH > 3,0.

3. Procédé d'obtention du chlorhydrate selon l'une des revendications 1 ou 2 **caractérisé en ce qu'**il consiste à dissoudre de la tritoqualine base dans un alcool, notamment un alcool aliphatique inférieur, ou dans un dérivé glycolé, en présence d'acide chlorhydrique jusqu'à obtenir une solution en état de sursaturation, puis à traiter ladite solution à l'aide d'un éther ajouté à ladite solution jusqu'à précipitation dudit chlorhydrate.

4. Procédé selon la revendication 3 **caractérisé en ce que** l'alcool est de l'éthanol.

5. Procédé selon la revendication 3 **caractérisé en ce que** le dérivé glycolé est le diméthyl glycol, le di(2-méthoxyéthyl)éther, ou le diéthylène glycol monométhyl éther.

6. Procédé selon la revendication 3 **caractérisé en ce que** ledit éther est l'éther éthylique.

7. Procédé selon la revendication 3 **caractérisé en ce que** ledit éther est le ter-butyl méthyl éther ou le tétrahydrofurane.

8. Procédé selon l'une quelconque des revendications 3 à 7 **caractérisé en ce que** ledit traitement se fait par ajouts successifs de fractions d'éther.

9. Procédé selon la revendication 8 **caractérisé en ce que**, avant d'effectuer un nouvel ajout d'éther, on attend que la solution ne soit plus opalescente.

10. Procédé selon l'une quelconque des revendications 3 à 9 **caractérisé en ce que** la proportion d'éther utilisée est de 4 volumes pour 1,5 volume d'alcool ou de dérivé glycolé permettant la dissolution de la tritoqualine.

## Patentansprüche

1. Tritoqualinhydrochlorid, **dadurch gekennzeichnet, dass** es in kristallisierter Form vorliegt.

2. Hydrochlorid nach Anspruch 1, **dadurch gekennzeichnet, dass** es in einer wässrigen Lösung löslich ist, bei pH < 3,0 hydrophil ist und bei pH > 3,0 als sehr feine Partikel ausfällt.

3. Verfahren zur Herstellung des Hydrochlorids nach einem der Ansprüche 1 oder 2, **dadurch gekennzeichnet, dass** es darin besteht, basisches Tritoqualin in einem Alkohol, insbesondere einem niedren aliphatischen Alkohol, oder einem Glykolderivat, in Gegenwart von Salzsäure solange aufzulösen, bis sich eine übersättigte Lösung ergibt, und anschließend diese Lösung mithilfe eines der Lösung hinzugegebenen Ethers bis zum Ausfällen des Hydrochlorids zu behandeln.

4. Verfahren nach Anspruch 3, **dadurch gekennzeichnet, dass** es sich beim Alkohol um Ethanol handelt.

5. Verfahren nach Anspruch 3, **dadurch gekennzeichnet, dass** das Glykolderivat Dimethylglykol, Di(2-methoxyethyl)ether oder Diethylenglykolmonomethylether ist.

6. Verfahren nach Anspruch 3, **dadurch gekennzeichnet, dass** es sich beim Ether um Ethylether handelt.

7. Verfahren nach Anspruch 3, **dadurch gekennzeichnet, dass** es sich beim Ether um Tert-Butylmethylether oder Tetrahydrofuran handelt.

8. Verfahren nach einem der Ansprüche 3 - 7, **dadurch gekennzeichnet, dass** die Behandlung durch aufeinanderfolgende Zugaben von Etherfraktionen erfolgt.

9. Verfahren nach Anspruch 8, **dadurch gekennzeichnet, dass** man vor einer weiteren Etherzugabe abwartet, bis die Lösung nicht mehr opaleszent ist.

10. Verfahren nach einem der Ansprüche 3 - 9, **dadurch gekennzeichnet, dass** der Ether im Volumenverhältnis 4 : 1,5 zum Volumen des Alkohols bzw. Ethers, der die Auflösung des Tritoqualins ermöglicht hat, verwendet wird.

## Claims

1. Tritoqualine hydrochloride **characterized in that** it is in crystalline form.

2. Hydrochloride according to claim 1 **characterized in that** it is soluble in aqueous solution and hydrophilic at pH < 3.0 and that it precipitates in the form of very fine particles at pH > 3.0.

3. Method for obtaining hydrochloride according to one of the claims 1 or 2, **characterized in that** this consists in dissolving tritoqualine base in an alcohol, especially a lower aliphatic alcohol or in a glycol derivative, in the presence of hydrochloric acid to form a solution in the supersaturation state, and then treating the solution with an ether added to the solution until precipitation of the hydrochloride.

4. Method according to claim 3 **characterized in that** the alcohol is ethanol.

5. Method according to claim 3 **characterized in that** the glycol derivative is dimethyl glycol, di(2-methoxyethyl) ether, or diethylene glycol monomethyl ether.

6. Method according to claim 3 **characterized in that** the ether is ethyl ether.

7. Method according to claim 3 **characterized in that** the ether is tert-butyl methyl ether or tetrahydrofuran.

8. Method according to any one of the claims 3 to 7 **characterized in that** the treatment is effected by successive additions of ether fractions.

9. Method according to claim 8 **characterized in that**, before performing a new addition of ether, time must elapse until the solution is no longer opalescent.

10. Method according to any one of the claims 3 to 9 **characterized in that** the proportion of ether used is 4 volumes per 1.5 volume of alcohol or glycol derivative allowing the dissolution of the tritoqualine.
